# EUROPEAN PATENT APPLICATION

(11) **EP 2 546 267 A1**
(43) Date of publication of application: **16.01.2013**
(21) Application number: 11173878.7
(22) Date of filing: 13.07.2011
(51) Int. Cl.: C07K 16/24, C12N 9/90, C12N 15/70

(54) **Bacterial host strain expressing recombinant DsbC**

(71) Applicant: UCB Pharma S.A., 1070 Brussels (BE)
(72) Inventor: The designation of the inventor has not yet been filed
(74) Representative: UCB Intellectual Property

(57) **Abstract**

The present invention provides a recombinant gram-negative bacterial cell comprising:
a. a recombinant polynucleotide encoding mutant DsbC; and
b. a polynucleotide sequence encoding an antibody or an antigen binding fragment thereof specific for human TNFα.

## Description

The invention relates to a recombinant bacterial host strain, particularly E. *coli.* The invention also relates to a method for producing a protein of interest in such a cell.

### Background of the invention

Bacterial cells, such as E. *coli,* are commonly used for producing recombinant proteins. There are many advantages to using bacterial cells, such as E. *coli,* for producing recombinant proteins particularly due to the versatile nature of bacterial cells as host cells allowing the gene insertion via plasmids. *E. coli* have been used to produce many recombinant proteins including human insulin.

Despite the many advantages to using bacterial cells to produce recombinant proteins, there can still be significant limitations including poor cell health phenotype.

Accordingly, there is still a need to provide new bacterial strains which provide advantageous means for producing recombinant proteins.

### Summary of the Invention

The present invention provides a recombinant gram-negative bacterial cell comprising:
a. a recombinant polynucleotide encoding a mutant DsbC; and
b. a polynucleotide sequence encoding an antibody or an antigen binding fragment thereof specific for human TNFα.

The gram-negative bacterial cell according to the present invention shows advantageous growth and protein production phenotypes.

The present invention also provides an expression vector comprising a recombinant polynucleotide encoding DsbC and an antibody or an antigen binding fragment thereof specific for human TNFα.

The present invention also provides a method for producing an antibody or an antigen binding fragment thereof specific for human TNFα comprising:
a. culturing a recombinant gram-negative bacterial cell as defined above in a culture medium under conditions effective to express the antibody or the antigen binding fragment thereof specific for human TNFα and the recombinant polynucleotide encoding DsbC; and
b. recovering the antibody or an antigen binding fragment thereof specific for human TNFα from the periplasm of the recombinant gram-negative bacterial cell and/or the culture medium.

### Brief Description of the Drawings

Figure 1 shows the construction of a vector for use in producing a cell according to an embodiment of the present invention.
Figure 2 shows shows the structure of a compound called CDP870 comprising a modified Fab fragment derived from antibody hTNF40 covalently linked via a cysteine residue to a lysyl-maleimide linker wherein each amino group on the lysyl residue has covalently attached to it a methoxy PEG residue wherein n is about 420.

### Brief Description of the Sequences

SEQ ID NO: 1 shows the amino acid sequence of CDRH1 of hTNF40.
SEQ ID NO: 2 shows the amino acid sequence of CDRH2 of hTNF40 which is a hybrid CDR wherein the C-terminal six amino acids are from the H2 CDR sequence of a human subgroup 3 germline antibody and the amino acid changes to the sequence resulting from this hybridisation are underlined as follows: WINTYIGEPI YADSVKG.
SEQ ID NO: 3 shows the amino acid sequence of CDRH3 of hTNF40.
SEQ ID NO: 4 shows the amino acid sequence of CDRL1 of hTNF40.
SEQ ID NO: 5 shows the amino acid sequence of CDRL2 of hTNF40.
SEQ ID NO: 6 shows the amino acid sequence of CDRL3 of hTNF40.
SEQ ID NO: 7 shows the amino acid sequence of CDRH2 of hTNF40.
SEQ ID NO: 8 shows the nucleotide and predicted amino acid sequence of the light chain variable region hTNF40-gL1.
SEQ ID NO: 9 shows the predicted amino acid sequence of the light chain variable region hTNF40-gL1.
SEQ ID NO: 10 shows the nucleotide and predicted amino acid sequence of the light chain variable region hTNF40-gL2.
SEQ ID NO: 11 shows the predicted amino acid sequence of the light chain variable region hTNF40-gL2.
SEQ ID NO: 12 shows the nucleotide and predicted amino acid sequence of the heavy chain variable region gh1hTNF40.4.
SEQ ID NO: 13 shows the predicted amino acid sequence of the heavy chain variable region gh1hTNF40.4.
SEQ ID NO:14 shows the nucleotide and predicted amino acid sequence of the heavy chain variable region gh3hTNF40.4.
SEQ ID NO:15 shows the predicted amino acid sequence of the heavy chain variable region gh3hTNF40.4.
SEQ ID NO: 16 shows the nucleotide sequence of a grafted anti-TNFα Fab heavy chain. SEQ ID NO: 17 shows the amino acid sequence of a grafted anti-TNFα Fab heavy chain. SEQ ID NO: 18 shows the nucleotide sequence of a grafted anti-TNFα Fab light chain. SEQ ID NO: 19 shows the amino acid sequence of a grafted anti-TNFα Fab light chain. SEQ ID NO: 20 shows the nucleotide sequence of a grafted anti-TNFα Fab heavy chain. SEQ ID NO: 21 shows the amino acid sequence of a grafted anti-TNFα Fab heavy chain. SEQ ID NO: 22 shows the sequence of the OmpA oligonucleotide adapter.
SEQ ID NO: 23 shows the oligonucleotide cassette encoding intergenic sequence 1 (IGS1) for *E. coli* Fab expression.
SEQ ID NO: 24 shows the oligonucleotide cassette encoding intergenic sequence 2 (IGS2) for *E. coli* Fab expression.
SEQ ID NO: 25 shows the oligonucleotide cassette encoding intergenic sequence 3 (IGS3) for *E. coli* Fab expression.
SEQ ID NO: 26 shows the oligonucleotide cassette encoding intergenic sequence 4 (IGS4) for *E. coli* Fab expression.
SEQ ID NO: 27 is the nucleotide sequence of his-tagged DsbC.
SEQ ID NO: 28 is the amino acid sequence of his-tagged DsbC.

### Detailed Description of the Preferred Embodiments of the Invention

The present invention will now be described in more detail.

The terms "protein" and "polypeptide" are used interchangeably herein, unless the context indicates otherwise. "Peptide" is intended to refer to 10 or less amino acids.

The terms "polynucleotide" includes a gene, DNA, cDNA, RNA, mRNA etc unless the context indicates otherwise.

As used herein, the term "comprising" in context of the present specification should be interpreted as "including".

The non-mutated cell or control cell in the context of the present invention means a cell of the same type as the recombinant gram-negative cell of the invention wherein the cell has not been modified to carry the recombinant polynucleotide sequence encoding DsbC and the polynucleotide sequence encoding an antibody or an antigen binding fragment thereof specific for human TNFα. For example, a non-mutated cell may be a wild-type cell and may be derived from the same population of host cells as the cells of the invention before modification to introduce any recombinant polynucleotide sequences.

The expressions "cell", "cell line", "cell culture" and "strain" are used interchangeably.

The term "isogenic" in the context of the present invention means that the cell comprises the same or substantially the same nucleic acid sequence(s) compared to wild-type cell except for the recombinant polynucleotide encoding mutant DsbC and the polynucleotide sequence encoding an antibody or an antigen binding fragment thereof specific for human TNFα (and optionally including other proteins according to the invention). In this embodiment the cell according to the present invention comprises no further non-naturally occurring or genetically engineered mutations.

In one embodiment wherein the polynucleotide sequence encoding mutant DsbC and/or the polynucleotide sequence encoding the antibody are inserted into the cell's genome, the cell according to the present invention may have substantially the same genomic sequence compared to the wild-type cell except for the polynucleotide sequence encoding DsbC and/or the polynucleotide sequence encoding the antibody taking into account any naturally occurring mutations which may occur. In one embodiment, wherein the polynucleotide sequence encoding DsbC and/or the polynucleotide sequence encoding the antibody are inserted into the cell's genome, the cell according to the present invention may have exactly the same genomic sequence compared to the wild-type cell except for the polynucleotide sequence encoding DsbC and/or the polynucleotide sequence encoding the antibody.

The polynucleotide encoding DsbC may be present on a suitable expression vector transformed into the cell and/or integrated into the host cell's genome. In the embodiment where the polynucleotide encoding DsbC is inserted into the host's genome, the cell of the present invention differs from a wild-type cell due to the inserted polynucleotide sequence encoding the DsbC. In this embodiment, the host cell's genome may be isogenic compared to a wild-type cell genome except for the recombinant polynucleotide sequence encoding DsbC. Preferably the polynucleotide encoding mutant DsbC is in an expression vector in the cell thereby causing minimal disruption to the host cell's genome.

The polynucleotide encoding the antibody or an antigen binding fragment thereof specific for human TNFα may be contained within a suitable expression vector transformed into the cell and/or integrated into the host cell's genome. In the embodiment where the polynucleotide encoding the antibody is inserted into the host's genome, the cell of the present invention differs from a wild-type cell due to the inserted polynucleotide sequence encoding the antibody. In this embodiment, the host cell's genome may be isogenic compared to a wild-type cell genome except for the polynucleotide sequence encoding the antibody. Preferably the polynucleotide encoding the protein of interest is in an expression vector in the cell thereby causing minimal disruption to the host cell's genome.

In one embodiment the recombinant the recombinant polynucleotide encoding Mutant DsbC and the polynucleotide encoding the antibody are inserted into the host's genome. In this embodiment, the cell of the present invention differs from a wild-type cell due to the inserted recombinant polynucleotide encoding mutant DsbC and the polynucleotide sequence encoding the antibody. In this embodiment, the host cell's genome may be isogenic compared to a wild-type cell genome except for the recombinant polynucleotide sequence encoding mutant DsbC and the polynucleotide sequence encoding the antibody.

In a preferred embodiment the recombinant polynucleotide encoding Mutant DsbC and the polynucleotide encoding the antibody are present in the same or different expression vectors in the cell thereby causing minimal disruption to the host cell's genome. In this embodiment the cell's genome may be substantially the same or exactly the same compared to the genome of a wild-type cell.

The term "wild-type" in the context of the present invention means a strain of a gram-negative bacterial cell as it may occur in nature or may be isolated from the environment, which does not carry any recombinant sequences or genetically engineered mutations. An example of a wild-type strain of *E. coli* is W3110, such as W3110 K-12 strain.

The present inventors have provided a recombinant gram-negative bacterial cell suitable for expressing an antibody or an antigen binding fragment thereof specific for human TNFα which comprises a recombinant polynucleotide encoding mutant DsbC.

DsbC is a prokaryotic protein found in the periplasm of *E.coli* which catalyzes the formation of disulphide bonds in *E.coli.* DsbC has an amino acid sequence length of 236 (including signal peptide) and a molecular weight of 25.6 KDa (UniProt No. POAEG6). DsbC was first identified in 1994 (Missiakas et al. The Escherichia coli dsbC (xprA) gene encodes a periplasmic protein involved in disulfide bond formation, The EMBO Journal vol 13, no 8, p2013-2020, 1994 and Shevchik et al. Characterization of DsbC, a periplasmic protein of Erwinia chrysanthemi and Escherichia coli with disulfide isomerase activity, The EMBO Jounral vol 13, no 8, p2007-2012, 1994).

DsbC is characterized by the present of an active site -CXXC- wherein XX represents GY. Mutants of DsbC include where XX are any two amino acids with the proviso that XX does not represent GF. In one embodiment X and X are independently selected from one of the twenty natural amino acids that occurring in proteins, for example selected from alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine and valine.

In one embodiment XX represents: TF, GF, HH, NY, SF, MF, VH, SH, RF, FA, GA, MA, GI, AV, PS, QA, SV, PR, PP, AL, PL, FL, TR, LL, VL, QL or LQ.

In one embodiment the DsbC employed in a mutant form, for example where the active site is mutated, in particular as described above.

In one embodiment the mutant has at least the biological activity of the wild type protein, for example as measured in an *in vitro* assay.

In one embodiment the mutant has at greater biological activity than the wild type protein, for example as measured in an *in vitro* assay.

In one embodiment the mutant DsbC consists of a DsbC protein with a mutated active site -CXXC- wherein XX represents TF, GF, HH, NY, SF, MF, VH, SH, RF, FA, GA, MA, GI, AV, PS, QA, SV, PR, PP, AL, PL, FL, TR, LL, VL, QL, LQ.

In one embodiment the mutation in DsbC consists of a mutation in the active site.

It is known to co-express proteins which catalyze the formation of disulphide bonds to improve protein expression in a host cell. WO98/56930 discloses a method for producing heterologous disulfide bond-containing polypeptides in bacterial cells wherein a prokaryotic disulfide isomerase, such as DsbC or DsbG is co-expressed with a eukaryotic polypeptide. US6673569 discloses an artificial operon comprising polynucleotides encoding each of DsbA, DsbB, DsbC and DsbD for use in producing a foreign protein. EP0786009 discloses a process for producing a heterologous polypeptide in bacteria wherein the expression of nucleic acid encoding DsbA or DsbC is induced prior to the induction of expression of nucleic acid encoding the heterologous polypeptide. However, there has not been previously provided a gram-negative bacterial cell comprising a recombinant polynucleotide encoding DsbC and a polynucleotide encoding an anti-TNFα antibody.

The cells according to the present invention comprise a recombinant polynucleotide encoding mutant DsbC. As used herein, a "recombinant polypeptide" refers to a protein that is constructed or produced using recombinant DNA technology. The polynucleotide sequence encoding DsbC with a mutated active site may be identical in all other ways to the endogenous sequence encoding DsbC found in bacterial cells. Alternatively, the recombinant polynucleotide sequence encoding DsbC is a mutated version of the wild-type DsbC sequence, for example having a restriction site removed, such as an EcoRI site, and/or a sequence encoding a his-tag. An examples of how to modified a DsbC sequence is shown in SEQ ID NO: 27, which encodes the his-tagged DsbC sequence shown in SEQ ID NO:28.

The present inventors have identified that the selection of the expression of recombinant polynucleotide encoding mutant DsbC in a bacterial cell, provides an improved host for expressing an antibody or an antigen binding fragment thereof specific for human TNFα. The cells provided by the present invention have improved cell health and growth phenotype compared to wild-type bacterial cells.

The cells according to the present invention exhibit improved antibody production yield compared to wild-type bacterial cells. The improved protein yield may be the rate of protein production and/or the duration of protein production from the cell. The improved protein yield may be the periplasmic protein yield and/or the supernatant protein yield. The recombinant bacterial cells may be capable of faster rate of production of the antibody and, therefore, the same quantity of a protein of interest may be produced in a shorter time compared to a non-mutated bacterial cell. The faster rate of production of a protein of interest may be especially significant over the initial period of growth of the cell, for example over the first 5, 10, 20 or 30 hours post induction of protein expression.

The cells according to the present invention preferably express a maximum yield in the periplasm and/or media of approximately 1.0g/L, 1. Sg/L, 1.8g/L, 2.0g/L, 2.4g/L, 2.5g/L, 3.Og/L, 3.5g/L or 4.0g/L of the antibody.

The skilled person would easily be able to test a candidate cell clone to see if it has the desired yield of a protein of interest using methods well known in the art including a fermentation method, ELISA and protein G HPLC. Suitable fermentation methods are described in Humphreys D P, et al. (1997). Formation of dimeric Fabs in E. coli: effect of hinge size and isotype, presence of interchain disulphide bond, Fab' expression levels, tail piece sequences and growth conditions. J. IMMUNOL. METH. 209: 193-202; Backlund E. Reeks D. Markland K. Weir N. Bowering L. Larsson G. Fedbatch design for periplasmic product retention in Escherichia coli, Journal Article. Research Support, Non-U.S. Gov't Journal of Biotechnology. 135(4):358-65, 2008 Jul 31; Champion KM. Nishihara JC. Joly JC. Arnott D. Similarity of the Escherichia coli proteome upon completion of different biopharmaceutical fermentation processes. [Journal Article] Proteomics. 1(9):1133-48, 2001 Sep; and Horn U. Strittmatter W. Krebber A. Knupfer U. Kujau M. Wenderoth R. Muller K. Matzku S. Pluckthun A. Riesenberg D. High volumetric yields of functional dimeric miniantibodies in Escherichia coli, using an optimized expression vector and high-cell-density fermentation under non-limited growth conditions, Journal Article. Research Support, Non-U.S. Gov't Applied Microbiology & Biotechnology. 46(5-6):524-32, 1996 Dec. The skilled person would also easily be able to test secreted protein to see if the protein is correctly folded using methods well known in the art, such as protein G HPLC, circular dichroism, NMR, X-Ray crystallography and epitope affinity measurement methods.

In one embodiment the cell according to the present invention also expresses one or more further proteins as follows:
- one or more proteins capable of facilitating protein folding, such as FkpA, Skp, SurA, PPiA and PPiD; and/or
- one or more protein capable of facilitating protein secretion or translocation, such as SecY, SecE, SecG, SecYEG, SecA, SecB, FtsY and Lep; and/or
- one or more proteins capable of facilitating disulphide bond formation, such as DsbA, DsbB, DsbD, DsbG.

One of more of the above proteins may be integrated into the cell's genome and/or inserted in an expression vector.

In one embodiment the cell according to the present invention does not comprise any recombinant polynucleotide encoding one or more of the following further proteins:
- one or more proteins capable of facilitating protein folding, such as FkpA, Skp, SurA, PPiA and PPiD;
- one or more protein capable of facilitating protein secretion or translocation, such as SecY, SecE, SecG, SecYEG, SecA, SecB, FtsY and Lep; and
- one or more proteins capable of facilitating disulphide bond formation, such as DsbA, DsbB, DsbD, DsbG.

In one embodiment the cell further comprises one or more of the following mutated genes:
a. a mutated spr gene;
b. a mutated Tsp gene, wherein the mutated Tsp gene encodes a Tsp protein having reduced protease activity or is a knockout mutated Tsp gene;
c. a mutated DegP gene encoding a DegP protein having chaperone activity and reduced protease activity;
d. a mutated ptr gene, wherein the mutated ptr gene encodes a Protease III protein having reduced protease activity or is a knockout mutated ptr gene; and/or
e. a mutated OmpT gene, wherein the mutated OmpT gene encodes a OmpT protein having reduced protease activity or is a knockout mutated OmpT gene.

In one embodiment the gram-negative bacterial cell according to the present invention does not carry a knockout mutated Tsp gene, such as being deficient in chromosomal Tsp.

In one embodiment the gram-negative bacterial cell according to the present invention comprises a mutated spr gene.

In one embodiment the gram-negative bacterial cell according to the present invention comprises a mutated Tsp gene, wherein the mutated Tsp gene encodes a Tsp protein having reduced protease activity or is a knockout mutated Tsp gene.

In one embodiment the gram-negative bacterial cell according to the present invention comprises a mutated Tsp gene, wherein the mutated Tsp gene encodes a Tsp protein having reduced protease activity or is a knockout mutated Tsp gene and a mutate spr gene and optionally a FkpA and/or Skp gene.

In one embodiment the gram-negative bacterial cell according to the present invention does not carry a knockout mutated ompT gene, such as being deficient in chromosomal ompT.

In one embodiment the gram-negative bacterial cell according to the present invention does not carry a knockout mutated degP gene, such as being deficient in chromosomal degP. In one embodiment the gram-negative bacterial cell according to the present invention does not carry a mutated degP gene.

In one embodiment the gram-negative bacterial cell according to the present invention does not carry a knockout mutated ptr gene, such as being deficient in chromosomal ptr.

In one embodiment the gram-negative bacterial cell according to the present invention does not carry a mutated spr gene.

Any suitable gram-negative bacterium may be used as the parental cell for producing the recombinant cell of the present invention. Suitable gram-negative bacterium include *Salmonella typhimurium, Pseudomonas fluorescens, Erwinia carotovora, Shigella, Klebsiella pneumoniae, Legionella pneumophila, Pseudomonas aeruginosa, Acinetobacter baumannii and E. coli.* Preferably the parental cell is *E. coli.* Any suitable strain of *E. coli* may be used in the present invention but preferably a wild-type W3110 strain, such as K-12 W3110, is used.

A drawback associated with strains previously created and used to express recombinant proteins involves the use of mutations of genes involved in cell metabolism and DNA replication such as, for example *phoA, fhuA, lac, rec, gal, ara, arg, thi* and *pro* in *E. coli* strains. These mutations may have many deleterious effects on the host cell including effects on cell growth, stability, recombinant protein expression yield and toxicity. Strains having one or more of these genomic mutations, particularly strains having a high number of these mutations, may exhibit a loss of fitness which reduces bacterial growth rate to a level which is not suitable for industrial protein production. Further, any of the above genomic mutations may affect other genes in *cis* and/or in *trans* in unpredictable harmful ways thereby altering the strain's phenotype, fitness and protein profile. Further, the use of heavily mutated cells is not generally suitable for producing recombinant proteins for commercial use, particularly therapeutics, because such strains generally have defective metabolic pathways and hence may grow poorly or not at all in minimal or chemically defined media.

In a preferred embodiment, the cells carry only the minimal mutations to introduce the recombinant polynucleotide encoding DsbC and the polynucleotide sequence encoding the antibody. In one embodiment wherein the polynucleotide sequence encoding DsbC and/or the polynucleotide sequence encoding the antibody are inserted into the cell's genome only minimal mutations are made to the cell's genome to introduce recombinant polynucleotide encoding DsbC and/or the antibody. In a further embodiment wherein the recombinant polynucleotide encoding DsbC and the polynucleotide encoding the antibody are present in the same or different expression vectors, the genome is preferably isogenic to a wild-type cell genome. The cells do not carry any other mutations which may have deleterious effects on the cell's growth and/or ability to express a protein of interest. Accordingly, one or more of the recombinant host cells of the present invention may exhibit improved protein expression and/or improved growth characteristics compared to cells comprising genetically engineered mutations to the genomic sequence. The cells provided by the present invention are also more suitable for use to produce therapeutic proteins compared to cells comprising disruptions to the cell genome.

In a preferred embodiment, the cell is isogenic to a wild-type *E. coli* cell except for the recombinant polynucleotide encoding mutant DsbC and the polynucleotide sequence encoding an antibody or an antigen binding fragment thereof specific for human TNFα. More preferably the cell according to the present invention is isogenic to an E. *coli* strain W3110 except for the recombinant polynucleotide encoding mutant DsbC and the polynucleotide sequence encoding an antibody or an antigen binding fragment thereof specific for human TNFα.

The cell provided by the present invention comprises a polynucleotide sequence encoding an antibody specific for TNFα. The antibody may be a multi-valent, multi-specific, humanized, fully human or chimeric antibody. The antibody can be from any species but is preferably derived from a monoclonal antibody, a human antibody, or a humanized fragment. The antibody can be derived from any class (e.g. IgG, IgE, IgM, IgD or IgA) or subclass of immunoglobulin molecule and may be obtained from any species including for example mouse, rat, shark, rabbit, pig, hamster, camel, llama, goat or human. Parts of the antibody fragment may be obtained from more than one species for example the antibody fragments may be chimeric. In one example the constant regions are from one species and the variable regions from another.

The antibody may comprise: a complete antibody molecule, having full length heavy and light chains; a fragment thereof, such as a VH, VL, VHH, Fab, modified Fab, Fab', F(ab')₂ or Fv fragment; a light chain or heavy chain monomer or dimer; a single chain antibody, e.g. a single chain Fv in which the heavy and light chain variable domains are joined by a peptide linker, Fab-Fv, or a dual specificity antibody, such as a Fab-dAb, as described in PCT/GB2008/003331. Similarly, the heavy and light chain variable regions may be combined with other antibody domains as appropriate.

The anti-TNFα antibody, is preferably an antibody derived from a mouse monoclonal antibody hTNF40 as described in WO01/094585 (the contents of which are incorporated herein by reference).

In a one embodiment the antibody having specificity for human TNFα, comprises a heavy chain wherein the variable domain comprises a CDR having the sequence shown in SEQ ID NO:1 for CDRH1, the sequence shown in SEQ ID NO:2 or SEQ ID NO:7 for CDRH2 or the sequence shown in SEQ ID N0:3 for CDRH3.

In one embodiment the antibody comprises a light chain wherein the variable domain comprises a CDR having the sequence shown in SEQ ID NO:4 for CDRL1, the sequence shown in SEQ ID NO:5 for CDRL2 or the sequence shown in SEQ ID NO:6 for CDRL3 .

The CDRs given in SEQ IDS NOS:1 and 3 to 7 referred to above are derived from a mouse monoclonal antibody hTNF40. However, SEQ ID NO:2 consists of a hybrid CDR. The hybrid CDR comprises part of heavy chain CDR2 from mouse monoclonal antibody hTNF40 (SEQ ID NO:7) and part of heavy chain CDR2 from a human group 3 germline V region sequence.

In one embodiment the antibody comprises a heavy chain wherein the variable domain comprises a CDR having the sequence shown in SEQ ID NO: 1 for CDRH1, the sequence shown in SEQ ID NO:2 or SEQ ID NO:7 for CDRH2 or the sequence shown in SEQ ID NO:3 for CDRH3 and a light chain wherein the variable domain comprises a CDR having the sequence shown in SEQ ID NO: 4 for CDRL1, the sequence shown in SEQ ID NO:5 for CDRL2 or the sequence shown in SEQ ID NO:6 for CDRL3.

In one embodiment the antibody comprises SEQ ID NO:1 for CDRH1, SEQ ID NO: 2 or SEQ ID N0:7 for CDRH2, SEQ ID N0:3 for CDRH3, SEQ ID N0:4 for CDRL1, SEQ ID NO:5 for CDRL2 and SEQ ID NO:6 for CDRL3. Preferably the antibody comprises SEQ ID NO:2 for CDRH2.

The antibody is preferably a CDR-grafted antibody molecule and typically the variable domain comprises human acceptor framework regions and non-human donor CDRs.

Preferably, the antibody comprises the light chain variable domain hTNF40-gL1 (SEQ ID N0:8) and the heavy chain variable domain gh3hTNF40.4 (SEQ ID N0:14).

Preferably the antibody is a Fab fragment. Preferably the Fab fragment has a heavy chain comprising or consisting of the sequence given as SEQ ID NO: 17 and a light chain comprising or consisting of the sequence given as SEQ ID NO:19. The amino acid sequences given in SEQ ID NO: 17 and SEQ ID NO: 19 are preferably encoded by the nucleotide sequences given in SEQ ID NO:16 and SEQ ID NO: 18, respectively.

Alternatively, it is preferred that the antibody is a modified Fab fragment wherein the modification is the addition to the C-terminal end of its heavy chain one or more amino acids to allow the attachment of an effector or reporter molecule. Preferably, the additional amino acids form a modified hinge region containing one or two cysteine residue to which the effector or reporter molecule may be attached. Such a modified Fab fragment preferably has a heavy chain comprising or consisting of the sequence given as SEQ ID NO:21 and the light chain comprising or consisting of the sequence given as SEQ ID NO: 19. The amino acid sequence given in SEQ ID NO:21 is preferably encoded by the nucleotide sequence given in SEQ ID NO:20.

The antibody may be a variant antibody, which has an improved affinity for TNFα. Such variants can be obtained by a number of affinity maturation protocols including mutating the CDRs (Yang et al., J. Mol. Biol., 254, 392-403, 1995), chain shuffling (Marks et al., Bio/Technology, 10, 779-783, 1992), use of mutator strains of *E*. *coli* (Low et al., J. Mol. Biol., 250, 359-368, 1996), DNA shuffling (Patten et al., Curr. Opin. Biotechnol., 8, 724-733, 1997), phage display (Thompson et al., J. Mol. Biol., 256, 77-88, 1996) and sexual PCR (Crameri et al., Nature, 391, 288-291, 1998). Vaughan *et al.* (*supra*) discusses these methods of affinity maturation.

The cell according to the present invention comprises DNA sequence encoding the antibody. Preferably, the DNA sequence encodes the heavy and the light chain of the antibody.

In one preferred embodiment, the DNA sequence encodes a light chain and comprises the sequence shown in SEQ ID NO:8 (hTNF40-gL1) or SEQ ID NO:10 (h-TNF-40-gL2) or a degenerate equivalent thereof

In an alternatively preferred embodiment, the DNA sequence encodes a heavy chain and comprises the sequence shown in SEQ ID NO:12 (gh1hTNF40.4) or SEQ ID N0:14 (gh3hTNF40.4) or a degenerate equivalent thereof.

Preferably the cell comprises the DNA sequence shown in SEQ ID NO: 20 and the DNA sequence shown in SEQ ID NO: 18.

The DNA sequence of the may comprise synthetic DNA, for instance produced by chemical processing, cDNA, genomic DNA or any combination thereof

The antibody preferably has a binding affinity of at least 0.85x10⁻¹⁰ M, more preferably at least 0.75x10⁻¹⁰ M and most preferably at least 0.5x10⁻¹⁰ M. (It is worth noting that the preferred humanised antibody, has an affinity of about 0.5x10⁻¹⁰M, which is better than the affinity of the murine monoclonal antibody from which it is derived. The murine antibody has an affinity of about 0.85x10⁻¹⁰M.)

The constant region domains of the antibody, if present, may be selected having regard to the proposed function of the antibody molecule, and in particular the effector functions which may be required. For example, the constant region domains may be human IgA, IgD, IgE, IgG or IgM domains. In particular, human IgG constant region domains may be used, especially of the IgG1 and IgG3 isotypes when the antibody molecule is intended for therapeutic uses and antibody effector functions are required. Alternatively, IgG2 and IgG4 isotypes may be used when the antibody molecule is intended for therapeutic purposes and antibody effector functions are not required, e.g. for simply blocking TUFα activity.

The antibody may be useful in the treatment of diseases or disorders including inflammatory diseases and disorders, immune disease and disorders, fibrotic disorders and cancers.

The term "inflammatory disease" or "disorder" and "immune disease or disorder" includes rheumatoid arthritis, psoriatic arthritis, still's disease, Muckle Wells disease, psoriasis, Crohn's disease, ulcerative colitis, SLE (Systemic Lupus Erythematosus), asthma, allergic rhinitis, atopic dermatitis, multiple sclerosis, vasculitis, Type I diabetes mellitus, transplantation and graft-versus-host disease.

The term "fibrotic disorder" includes idiopathic pulmonary fibrosis (IPF), systemic sclerosis (or scleroderma), kidney fibrosis, diabetic nephropathy, IgA nephropathy, hypertension, end-stage renal disease, peritoneal fibrosis (continuous ambulatory peritoneal dialysis), liver cirrhosis, age-related macular degeneration (ARMD), retinopathy, cardiac reactive fibrosis, scarring, keloids, burns, skin ulcers, angioplasty, coronary bypass surgery, arthroplasty and cataract surgery.

The term "cancer" includes a malignant new growth that arises from epithelium, found in skin or, more commonly, the lining of body organs, for example: breast, ovary, prostate, lung, kidney, pancreas, stomach, bladder or bowel. Cancers tend to infiltrate into adjacent tissue and spread (metastasise) to distant organs, for example: to bone, liver, lung or the brain.

The cell may also comprise further polynucleotide sequences encoding one or more further proteins of interest.

The recombinant gram-negative bacterial cell according to the present invention may be produced by any suitable means.

The skilled person knows suitable techniques which may be used to insert the recombinant polynucleotide encoding mutant DsbC and the polynucleotide encoding the antibody. The recombinant polynucleotide encoding DsbC and/or the polynucleotide encoding the antibody may be integrated into the cell's genome using a suitable vector such as pK03, (Link et al., 1997, Journal of Bacteriology, 179, 6228-6237).

Alternatively or additionally, the recombinant polynucleotide encoding mutant DsbC and/or the polynucleotide encoding the antibody may be non-integrated in a recombinant expression cassette. In one embodiment an expression cassette is employed in the present invention to carry the polynucleotide encoding mutant DsbC and/or the polynucleotide encoding the antibody which typically comprises a protein coding sequence encoding mutant DsbC, one or more protein coding sequences encoding the antibody and one or more regulatory expression sequences. The one or more regulatory expression sequences may include a promoter. The one or more regulatory expression sequences may also include a 3' untranslated region such as a termination sequence. Suitable promoters are discussed in more detail below.

In one embodiment, the cell according to the present invention comprises one or more expression vectors, such as plasmid. The vector preferably comprises one or more of the expression cassettes as defined above. The cell preferably comprises a expression vector comprising DNA encoding an antibody or an antigen binding fragment thereof specific for human TNFα as described above. Preferably the expression vector comprises a polynucleotide sequence encoding a light chain and a polynucleotide sequence encoding a heavy chain of the antibody.

In a preferred embodiment, the expression vector is an *E. coli* expression vector.

In one embodiment the polynucleotide sequence encoding the antibody and the polynucleotide encoding mutant DsbC are inserted into separate expression vectors.

For production of products comprising both heavy and light chains, the cell line may be transfected with two vectors, a first vector encoding a light chain polypeptide and a second vector encoding a heavy chain polypeptide. Alternatively, a single vector may be used, the vector including sequences encoding light chain and heavy chain polypeptides.

Alternatively, the polynucleotide sequence encoding the antibody and the polynucleotide encoding DsbC are inserted into one vector. Preferably the vector comprises the sequences encoding the light and heavy chain polypeptides of the antibody.

The present invention also provides an expression vector comprising a recombinant polynucleotide encoding mutant DsbC and an antibody or an antigen binding fragment thereof specific for human TNFα. The expression vector is a multi-cistronic vector comprising the polynucleotide sequence encoding DsbC and the polynucleotide sequence encoding the antibody.

The multicistronic vector may be produced by an advantageous cloning method which allows repeated sequential cloning of polynucleotide sequences into a vector. The method uses compatible cohesive ends of a pair of restrictions sites, such as the "AT" ends of *Ase I* and *Nde I* restriction sites. A polynucleotide sequence comprising a coding sequence and having compatible cohesive ends, such as a *AseI-NdeI* fragment, may be cloned into a restrictions site in the vector, such as *Nde I.* The insertion of the polynucleotide sequence destroys the 5' restriction site but creates a new 3' restriction site, such as *NdeI,* which may then be used to insert a further polynucleotide sequence comprising compatible cohesive ends. The process may then be repeated to insert further sequences. Each polynucleotide sequence inserted into the vector comprises non-coding sequence 3' to the stop codon which may comprise an Ssp I site for screening, a Shine Dalgarno ribosome binding sequence, an A rich spacer and an *NdeI* site encoding a start codon.

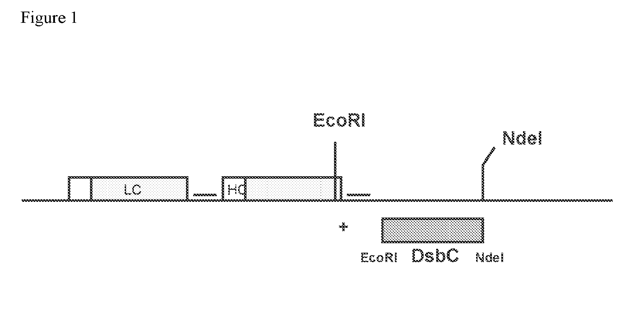

A diagrammatic representation of the creation of a vector comprising a polynucleotide sequence encoding a light chain of an antibody (LC), a heavy chain of an antibody (HC), a mutant DsbC polynucleotide sequence and a further polynucleotide sequence is shown in Figure 1.

The cell according to the present invention preferably comprises an expression vector as defined above.

In the embodiment wherein the cell also expresses one or more further proteins as follows:
- one or more proteins capable of facilitating protein folding, such as FkpA, Skp, SurA, PPiA and PPiD; and/or
- one or more protein capable of facilitating protein secretion or translocation, such as SecY, SecE, SecG, SecYEG, SecA, SecB, FtsY and Lep; and/or
- one or more proteins capable of facilitating disulphide bond formation, such as DsbA, DsbB, DsbD, DsbG;
the one or more further protein may be expressed from one or more polynucleotides inserted into the same vector as the polynucleotide encoding a mutated DsbC and/or the polynucleotide sequence encoding the antibody. Alternatively the one or more polynucleotides may be inserted into separate vectors.

The expression vector may be produced by inserting one or more expression cassettes as defined above into a suitable vector. Alternatively, the regulatory expression sequences for directing expression of the polynucleotide sequence may be contained in the expression vector and thus only the encoding region of the polynucleotide may be required to complete the expression vector.

The polynucleotide encoding a mutant DsbC and/or the polynucleotide encoding the antibody is suitably inserted into a replicable vector, typically an autonomously replicating expression vector, for expression in the cell under the control of a suitable promoter for the cell. Many vectors are known in the art for this purpose and the selection of the appropriate vector may depend on the size of the nucleic acid and the particularly cell type.

Examples of expression vectors which may be employed to transform the host cell with a polynucleotide according to the invention include:
- a plasmid, such as pBR322 or pACYC184, and/or
- a viral vector such as bacterial phage
- a transposable genetic element such as a transposon

Such expression vectors usually comprise a plasmid origin of DNA replication, an antibiotic selectable marker, a promoter and transcriptional terminator separated by a multicloning site (expression cassette) and a DNA sequence encoding a ribosome binding site.

The promoters employed in the present invention can be linked to the relevant polynucleotide directly or alternatively be located in an appropriate position, for example in a vector such that when the relevant polypeptide is inserted the relevant promoter can act on the same. In one embodiment the promoter is located before the encoding portion of the polynucleotide on which it acts, for example a relevant promoter before each encoding portion of polynucleotide. "Before" as used herein is intended to imply that the promoter is located at the 5 prime end in relation to the encoding polynucleotide portion.

The promoters may be endogenous or exogenous to the host cells. Suitable promoters include Lac, tac, trp, PhoA, Ipp, Arab, Tet and T7.

One or more promoters employed may be inducible promoters. In the embodiment wherein the polynucleotide encoding DsbC and the polynucleotide encoding the antibody are inserted into one vector, the nucleotide sequences encoding mutant DsbC and the antibody may be under the control of a single promoter or separate promoters. In the embodiment wherein the nucleotide sequences encoding mutant DsbC and the antibody are under the control of separate promoters, the promoter may be independently inducible promoters.

Promoters for use in bacterial systems also generally contain a Shine-Dalgamo (S.D.) sequence operably linked to the DNA encoding the polypeptide of interest. The promoter can be removed from the bacterial source DNA by restriction enzyme digestion and inserted into the vector containing the desired DNA.

In the embodiments of the present invention wherein the polynucleotide sequence encoding the antibody comprises two or more encoding sequences for an antibody light chain and antibody heavy chain, the polynucleotide sequence may comprise one or more internal ribosome entry site (IRES) sequences which allows translation initiation in the middle of an mRNA. An IRES sequence may be positioned between encoding polynucleotide sequences to enhance separate translation of the mRNA to produce the encoded polypeptide sequences.

The expression vector preferably also comprises a dicistronic message for producing the antibody or antigen binding fragment thereof as described in WO 03/048208 or WO2007/039714 (the contents of which are incorporated herein by reference). Preferably the upstream cistron contains DNA coding for the light chain of the antibody and the downstream cistron contains DNA coding for the corresponding heavy chain, and the dicistronic intergenic sequence (IGS) preferably comprises a sequence selected from IGS1 (SEQ ID NO: 23), IGS2 (SEQ ID NO: 24), IGS3 (SEQ ID NO: 25) and IGS4 (SEQ ID NO: 26).

The terminators may be endogenous or exogenous to the host cells. A suitable terminator is rrnB.

Further suitable transcriptional regulators including promoters and terminators and protein targeting methods may be found in "Strategies for Achieving High-Level Expression of Genes in Escherichia coli" Savvas C. Makrides, Microbiological Reviews, Sept 1996, p 512-538.

The mutant DsbC polynucleotide inserted into the expression vector preferably comprises the nucleic acid encoding the DsbC signal sequence and the DsbC coding sequence. The vector preferably contains a nucleic acid sequence that enables the vector to replicate in one or more selected host cells, preferably to replicate independently of the host chromosome. Such sequences are well known for a variety of bacteria.

In one embodiment the mutant DsbC and/or the protein of interest comprises a histidine-tag at the N-terminus and/or C-terminus.

The antibody molecule may be secreted from the cell or targeted to the periplasm by suitable signal sequences. Alternatively, the antibody molecules may accumulate within the cell's cytoplasm. Preferably the antibody molecule is targeted to the periplasm.

The polynucleotide encoding the antibody may be expressed as a fusion with another polypeptide, preferably a signal sequence or other polypeptide having a specific cleavage site at the N-terminus of the mature polypeptide. The heterologous signal sequence selected should be one that is recognized and processed by the host cell. For prokaryotic host cells that do not recognize and process the native or a eukaryotic polypeptide signal sequence, the signal sequence is substituted by a prokaryotic signal sequence. Suitable signal sequences include OmpA, PhoA, LamB, PelB, DsbA and DsbC. In an embodiment where the cell comprises a polynucleotide sequence encoding a heavy chain of the antibody and a polynucleotide sequence encoding a light chain of the antibody, each polynucleotide may comprise a signal sequence, such as OmpA.

Construction of suitable vectors containing one or more of the above-listed components employs standard ligation techniques. Isolated plasmids or DNA fragments are cleaved, tailored, and re-ligated in the form desired to generate the plasmids required. General methods by which the vectors may be constructed, transfection methods and culture methods are well known to those skilled in the art. In this respect, reference is made to "Current Protocols in Molecular Biology", 1999, F. M. Ausubel (ed), Wiley Interscience, New York and the Maniatis Manual produced by Cold Spring Harbor Publishing.

Standard techniques of molecular biology may be used to prepare DNA sequences coding for the antibody. Desired DNA sequences may be synthesised completely or in part using oligonucleotide synthesis techniques. Site-directed mutagenesis and polymerase chain reaction (PCR) techniques may be used as appropriate.

Embodiments of the invention described herein with reference to the polynucleotide apply equally to alternative embodiments of the invention, for example vectors, expression cassettes and/or host cells comprising the components employed therein, as far as the relevant aspect can be applied to same.

The present invention also provides a method for producing an antibody or an antigen binding fragment thereof specific for human TNFα comprising:
a. culturing a recombinant gram-negative bacterial cell as defined above in a culture medium under conditions effective to express the antibody or the antigen binding fragment thereof specific for human TNFα and the recombinant polynucleotide encoding mutant DsbC; and
b. recovering the antibody or an antigen binding fragment thereof specific for human TNFα from the periplasm of the recombinant gram-negative bacterial cell and/or the culture medium.

The gram negative bacterial cell and antibody preferably employed in the method of the present invention are described in detail above.

The recombinant polynucleotide encoding mutant DsbC and the polynucleotide encoding the antibody may be incorporated into the host cell using any suitable means known in the art. As discussed above, typically the polynucleotide encoding mutant DsbC and the polynucleotide encoding the antibody are incorporated as part of the same or separate expression vectors which are transformed into the cell.

The polynucleotide encoding mutant DsbC and the polynucleotide encoding the antibody can be transformed into a cell using standard techniques, for example employing rubidium chloride, PEG or electroporation.

The method according to the present invention may also employ a selection system to facilitate selection of stable cells which have been successfully transformed with the polynucleotide encoding the protein of interest. The selection system typically employs co-transformation of a polynucleotide sequence encoding a selection marker. In one embodiment, each polynucleotide transformed into the cell further comprises a polynucleotide sequence encoding one or more selection markers. Accordingly, the transformation of the polynucleotides encoding DsbC and the antibody and the one or more polynucleotides encoding the marker occurs together and the selection system can be employed to select those cells which produce the desired proteins.

Cells able to express the one or more markers are able to survive/grow/multiply under certain artificially imposed conditions, for example the addition of a toxin or antibiotic, because of the properties endowed by the polypeptide/gene or polypeptide component of the selection system incorporated therein (e.g. antibiotic resistance). Those cells that cannot express the one or more markers are not able to survive/grow/multiply in the artificially imposed conditions. The artificially imposed conditions can be chosen to be more or less vigorous, as required.

Any suitable selection system may be employed in the present invention. Typically the selection system may be based on including in the vector one or more genes that provides resistance to a known antibiotic, for example a tetracycline, chloramphenicol, kanamycin or ampicillin resistance gene. Cells that grow in the presence of a relevant antibiotic can be selected as they express both the gene that gives resistance to the antibiotic and the desired protein.

An inducible expression system or a constitutive promoter may be used in the present invention to express the antibody and/or the DsbC. Suitable inducible expression systems and constitutive promoters are well known in the art.

In one embodiment wherein the polynucleotide encoding DsbC and the polynucleotide encoding the antibody are under the control of the same or separate inducible promoters, the expression of the polynucleotide sequence encoding the antibody and the recombinant polynucleotide encoding DsbC is induced by adding an inducer to the culture medium.

Any suitable medium may be used to culture the transformed cell. The medium may be adapted for a specific selection system, for example the medium may comprise an antibiotic, to allow only those cells which have been successfully transformed to grow in the medium.

The cells obtained from the medium may be subjected to further screening and/or purification as required. The method may further comprise one or more steps to extract and purify the protein of interest as required.

The antibody may be recovered and purified from the strain, including from the cytoplasm, periplasm, or supernatant. Suitable methods include fractionation on immunoaffnity or ion-exchange columns; ethanol precipitation; reversed-phase HPLC; hydrophobic-interaction chromatography; chromatography on silica; chromatography on an ion-exchange resin such as S-SEPHAROSE and DEAE; chromatofocusing; ammonium-sulfate precipitation; and gel filtration.

In one embodiment the method further comprises separating the antibody from mutant DsbC.

Antibodies may be suitably separated from the culture medium and/or cytoplasm extract and/or periplasm extract by conventional antibody purification procedures such as, for example, protein A-Sepharose, protein G chromatography, protein L chromatograpy, thiophilic, mixed mode resins, Histag, FLAGTag, hydroxylapatite chromatography, gel electrophoresis, dialysis, affinity chromatography, Ammonium Sulphate, ethanol or PEG fractionation/precipitation, ion exchange membranes, expanded bed adsorption chromatography (EBA) or simulated moving bed chromatography.

The method may also include a further step of measuring the quantity of expression of the protein of interest and selecting cells having high expression levels of the protein of interest.

One or more method steps described herein may be performed in combination in a suitable container such as a bioreactor.

After expression, the antibody may be further processed, for example by conjugation to another entity such as an effector molecule. Accordingly, the method according to the present invention may comprise a further step of attaching an effector molecule to the antibody.

The term effector molecule as used herein includes, for example, antineoplastic agents, drugs, toxins (such as enzymatically active toxins of bacterial or plant origin and fragments thereof e.g. ricin and fragments thereof) biologically active proteins, for example enzymes, other antibody or antibody fragments, synthetic or naturally occurring polymers, nucleic acids and fragments thereof e.g. DNA, RNA and fragments thereof, radionuclides, particularly radioiodide, radioisotopes, chelated metals, nanoparticles and reporter groups such as fluorescent compounds or compounds which may be detected by NMR or ESR spectroscopy. Effector molecular may be attached to the antibody or fragment thereof by any suitable method, for example an antibody fragment may be modified to attach at least one effector molecule as described in WO05/003171 or WO05/003170 (the contents of which are incorporated herein by reference). WO05/003171 or WO05/003170 also describe suitable effector molecules.

The antibody may have a macrocycle, for chelating a heavy metal atom, or a toxin, such as ricin, attached to it by a covalent bridging structure. Alternatively, procedures of recombinant DNA technology may be used to produce an antibody molecule in which the Fc fragment (CH2, CH3 and hinge domains), the CH2 and CH3 domains or the CH3 domain of a complete immunoglobulin molecule has (have) been replaced by, or has attached thereto by peptide linkage, a functional non-immunoglobulin protein, such as an enzyme or toxin molecule. In the embodiment wherein the antibody is a modified Fab fragment having at the C-terminal end of its heavy chain one or more amino acids to allow attachment of an effector or reporter molecule, the additional amino acids preferably form a modified hinge region containing one or two cysteine residues to which the effector or reporter molecule may be attached.

A preferred effector group is a polymer molecule, which may be attached to the modified Fab fragment to increase its half-life *in vivo.*

The polymer molecule may, in general, be a synthetic or a naturally occurring polymer, for example an optionally substituted straight or branched chain polyalkylene, polyalkenylene or polyoxyalkylene polymer or a branched or unbranched polysaccharide, e.g. a homo- or hetero- polysaccharide.

Particular optional substituents which may be present on the above-mentioned synthetic polymers include one or more hydroxy, methyl or methoxy groups. Particular examples of synthetic polymers include optionally substituted straight or branched chain poly(ethyleneglycol), poly(propyleneglycol) poly(vinylalcohol) or derivatives thereof, especially optionally substituted poly(ethyleneglycol) such as methoxypoly(ethyleneglycol) or derivatives thereof Particular naturally occurring polymers include lactose, amylose, dextran, glycogen or derivatives thereof "Derivatives" as used herein is intended to include reactive derivatives, for example thiol-selective reactive groups such as maleimides and the like. The reactive group may be linked directly or through a linker segment to the polymer. It will be appreciated that the residue of such a group will in some instances form part of the product as the linking group between the antibody fragment and the polymer.

The size of the polymer may be varied as desired, but will generally be in an average molecular weight range from 500Da to 50000Da, preferably from 5000 to 4000Da and more preferably from 25000 to 4000Da. The polymer size may in particular be selected on the basis of the intended use of the product. Thus, for example, where the product is intended to leave the circulation and penetrate tissue, for example for use in the treatment of a tumour, it may be advantageous to use a small molecular weight polymer, for example with a molecular weight of around 5000Da. For applications where the product remains in the circulation, it may be advantageous to use a higher molecular weight polymer, for example having a molecular weight in the range from 25000Da to 40000Da.

Particularly preferred polymers include a polyalkylene polymer, such as a poly(ethyleneglycol) or, especially, a methoxypoly(ethyleneglycol) or a derivative thereof, and especially with a molecular weight in the range from about 25000Da to about 40000Da.

Each polymer molecule attached to the modified antibody fragment may be covalently linked to the sulphur atom of a cysteine residue located in the fragment. The covalent linkage will generally be a disulphide bond or, in particular, a sulphur-carbon bond.

Where desired, the antibody fragment may have one or more effector or reporter molecules attached to it. The effector or reporter molecules may be attached to the antibody fragment through any available amino acid side-chain or terminal amino acid functional group located in the fragment, for example any free amino, imino, hydroxyl or carboxyl group. One or more effector or reporter molecules may be attached to an amino acid at or towards the C-terminal end of the heavy chain and/or the light chain of the antibody.

An activated polymer may be used as the starting material in the preparation of polymer-modified antibody fragments as described above. The activated polymer may be any polymer containing a thiol reactive group such as an a-halocarboxylic acid or ester, e.g. iodoacetamide, an imide, e.g. maleimide, a vinyl sulphone or a disulphide. Such starting materials may be obtained commercially (for example from Shearwater Polymers Inc., Huntsville, AL, USA) or may be prepared from commercially available starting materials using conventional chemical procedures.

As regards attaching poly(ethyleneglycol) (PEG) moieties, reference is made to "Poly(ethyleneglycol) Chemistry, Biotechnical and Biomedical Applications", 1992, J. Milton Harris (ed), Plenum Press, New York, "Poly(ethyleneglycol) Chemistry and Biological Applications", 1997, J. Milton Harris and S. Zalipsky (eds), American Chemical Society, Washington DC and "Bioconjugation Protein Coupling Techniques for the Biomedical Sciences", 1998, M. Aslam and A. Dent, Grove Publishers, New York.

Where it is desired to obtain an antibody fragment linked to an effector or reporter molecule, this may be prepared by standard chemical or recombinant DNA procedures in which the antibody fragment is linked either directly or via a coupling agent to the effector or reporter molecule either before or after reaction with the activated polymer as appropriate. Particular chemical procedures include, for example, those described in WO 93/62331, WO 92/22583, WO 90,195 and WO 89/1476. Alternatively, where the effector or reporter molecule is a protein or polypeptide the linkage may be achieved using recombinant DNA procedures, for example as described in WO 86/01533 and EP-A-0392745.

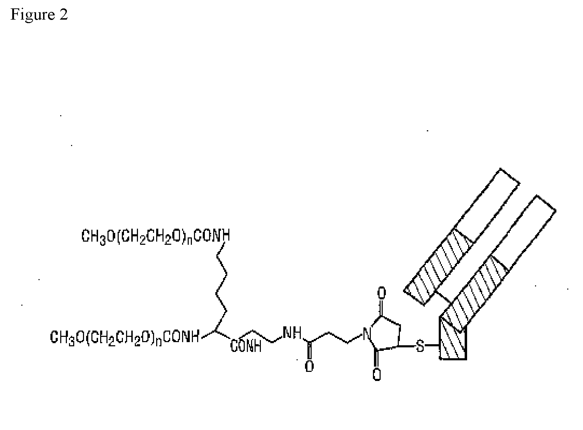

Preferably, the modified Fab fragment provided by the method of the present invention is PEGylated (i.e. has PEG (poly(ethyleneglycol)) covalently attached thereto) according to the method disclosed in EP-A-0948544. Preferably the antibody is a PEGylated modified Fab fragment as shown in Figure 2. As shown in Figure 2, the modified Fab fragment has attached to one of the cysteine residues at the C-terminal end of the modified hinge region of the heavy chain a lysyl-maleimide-derived group wherein each of the two amino groups of the lysyl residue has covalently linked to it a methoxypoly(ethyleneglycol) residue having a molecular weight of about 20,000 Da, such that the total average molecular weight of the methoxypoly(ethyleneglycol) residues is about 40,000Da, more preferably the lysyl-maleimide-derived group is [1-[[[2-[[3-(2,5-dioxo-1-pyrrolidinyl)-1-oxopropyl]amino]ethyl]amino]-carbonyl]-1,5-pentanediyl]bis(iminocarbonyl). A lysine residue is covalently linked to the maleimide group. To each of the amine groups on the lysine residue is attached a methoxypoly(ethyleneglycol) polymer having a molecular weight of approximately 20,000 Da. The total molecular weight of the entire effector molecule is therefore approximately 40,000 Da.

Accordingly, the method according to the present invention preferably comprises attaching to one of the cysteine residues at the C-terminal end of the heavy chain a lysyl-maleimide group wherein each amino group of the lysyl residue has covalently linked to it a methoxypoly(ethyleneglycol) residue having a molecular weight of about 20,000 Da.

Preferably, in the compound shown in Figure 2, the heavy chain of the antibody part has the sequence given as SEQ ID NO:21 and the light chain has the sequence given in SEQ ID N0:19. This compound is referred to herein as CDP870.

In one embodiment one or more *E.coli* host proteins that in the wild type are known to co-purify with the antibody during purification are selected for genetic modification, as described in Humphreys et al. "Engineering of Escherichia coli to improve the purification of periplasmic Fab' fragments: changing the pI of the chromosomally encoded PhoS/PstS protein", Protein Expression and Purification 37 (2004) 109-118 and WO04/035792 (the contents of which are incorporated herein by reference). The use of such modified host proteins improves the purification process for proteins of interest, especially antibodies, produced in *E.col* by altering the physical properties of selected *E.coli* proteins so they no longer co-purify with the recombinant antibody. Preferably the *E.coli* protein that is altered is selected from one or more of Phosphate binding protein (PhoS/PstS), Dipeptide binding protein (DppA), Maltose binding protein (MBP) and Thioredoxin.

In one embodiment a physical property of a contaminating host protein is altered by the addition of an amino acid tag to the C-terminus or N-terminus. In a preferred embodiment the physical property that is altered is the isoelectric point and the amino acid tag is a poly-aspartic acid tag attached to the C-terminus. In one embodiment the *E.coli* proteins altered by the addition of said tag are Dipeptide binding protein (DppA), Maltose binding protein (MBP), Thioredoxin and Phosphate binding protein (PhoS/PstS). In one specific embodiment the pI of the *E.coli* Phosphate binding protein (PhoS/PstS) is reduced from 7.2 to 5.1 by the addition of a poly-aspartic acid tag (polyD), containing 6 aspartic acid residues to the C-terminus.

Also preferred is the modification of specific residues of the contaminating *E.coli* protein to alter its physical properties, either alone or in combination with the addition of N or C terminal tags. Such changes can include insertions or deletions to alter the size of the protein or amino acid substitutions to alter pI or hydrophobicity. In one embodiment these residues are located on the surface of the protein. In a preferred embodiment surface residues of the PhoS protein are altered in order to reduce the pI of the protein. Preferably residues that have been implicated to be important in phosphate binding (Bass, US5,304,472) are avoided in order to maintain a functional PhoS protein. Preferably lysine residues that project far out of the surface of the protein or are in or near large groups of basic residues are targeted. In one embodiment, the PhoS protein has a hexa poly-aspartic acid tag attached to the C-terminus whilst surface residues at the opposite end of the molecule are targeted for substitution. Preferably selected lysine residues are substituted for glutamic acid or aspartic acid to confer a greater potential pI change than when changing neutral residues to acidic ones. The designation for a substitution mutant herein consists of a letter followed by a number followed by a letter. The first letter designates the amino acid in the wild-type protein. The number refers to the amino acid position where the amino acid substitution is being made, and the second letter designates the amino acid that is used to replace the wild-type amino acid. In preferred mutations of PhoS in the present invention lysine residues (K) 275, 107, 109, 110, 262, 265, 266, 309, 313 are substituted for glutamic acid (E) or glutamine (Q), as single or combined mutations, in addition lysine(K)318 may be substituted for aspartic acid (D) as a single or combined mutation. Preferably the single mutations are K262E, K265E and K266E. Preferably the combined mutations are K265/266E and K110/265/266E. More preferably, all mutations are combined with the polyaspartic acid (polyD) tag attached at the C-terminus and optionally also with the K318D substitution. In a preferred embodiment the mutations result in a reduction in pI of at least 2 units. Preferably the mutations of the present invention reduce the pI of PhoS from 7.2 to between about 4 and about 5.5. In one embodiment of the present invention the pI of the PhoS protein of *E.coli* is reduced from 7.2 to about 4.9, about 4.8 and about 4.5 using the mutations polyD K318D, polyD K265/266E and polyD K110/265/266E respectively.

### Methods

### General methods that may be employed to prepare cell lines according to the invention: A dicistronic message encoding an anti-TNFα antibody

A dicistronic message was created of the anti-TNFα Fab' fragment described in WO01/94585. The upstream cistron encoded the light chain of the antibody (SEQ ID NO: 19) whilst the downstream cistron encoded the heavy chain of the antibody (SEQ ID NO: 21). A DNA sequence encoding the OmpA signal peptide was fused to the 5' end of the DNA coding for each of the light chain and the heavy chain to allow efficient secretion to the periplasm. The intergenic sequence (IGS) 2 was used as shown in SEQ ID NO: 24.

### Cell Lines

For all experiments the *E.coli* cell line W3110 was used as the wild-type cell line.

### Generation of plasmid for Fab' and DsbC expression

A plasmid was constructed containing both the heavy and light chain sequences of an anti-TNF Fab' and the sequence encoding DsbC (SEQ ID N0:27).

Plasmid pDPH358 (pTTO 40.4 CDP870 IGS2), an expression vector for the CDP870 Fab' (an anti-TNF Fab') and DsbC (a periplasmic polypeptide), was constructed using conventional restriction cloning methodologies which can be found in Sambrook et al 1989, Molecular cloning: a laboratory manual. CSHL press, N.Y. The plasmid pDPH358 contained the following features; a strong *tac* promoter and *lac* operator sequence. As shown in Figure 1, the plasmid contained a unique EcoRI restriction site after the coding region of the Fab' heavy chain, followed by a non-coding sequence and then a unique NdeI restriction site. The DsbC gene was PCR cloned using W3110 crude chromosomal DNA as a template such that the PCR product encoded for a 5' EcoRI site followed by a strong ribosome binding, followed by the native start codon, signal sequence and mature sequence of DsbC, terminating in a C-terminal His tag and finally a non-coding NdeI site. The EcoRI-NdeI PCR fragment was restricted and ligated into the expression vector such that all three polypeptides: Fab' light chain, Fab' heavy chain and DsbC were encoded on a single polycistronic mRNA.

The Fab light chain, heavy chain genes and DcbC gene were transcribed as a single polycistronic message. DNA encoding the signal peptide from the *E. coli* OmpA protein was fused to the 5' end of both light and heavy chain gene sequences, which directed the translocation of the polypeptides to the *E. coli* periplasm. Transcription was terminated using a dual transcription terminator *rrnB t1t2.* The *lacIq* gene encoded the constitutively expressed Lac I repressor protein. This repressed transcription from the tac promoter until de-repression was induced by the presence of allolactose or IPTG. The origin of replication used was p15A, which maintained a low copy number. The plasmid contained a tetracycline resistance gene for antibiotic selection.

### Expression of anti-TNF Fab' and DsbC in E.coli

### Expression of anti-TNFFab ' and DsbC

The *E.coli* W3110 cell strain was transformed with the plasmid generated above.

The transformation of the strains was carried out using the method found in Chung C.T et al Transformation and storage of bacterial cells in the same solution. PNAS 86:2172-2175 (1989).

### Expression of anti-TNFFab '

The *E.coli* W3110 cell strain was transformed with plasmid pMXE117 (pTTO CDP870 or 40.4 IGS2), an expression vector for the anti-TNFα Fab', which was constructed using conventional restriction cloning methodologies which can be found in Sambrook et al 1989, Molecular cloning: a laboratory manual. CSHL press, N.Y. The plasmid pMXE117 (pTTO CDP870 or 40.4 IGS2) contained the following features; a strong *tac* promoter and *lac* operator sequence. The Fab light and heavy chain genes were transcribed as a single dicistronic message. DNA encoding the signal peptide from the *E. coli* OmpA protein was fused to the 5' end of both light and heavy chain gene sequences, which directed the translocation of the polypeptides to the *E. coli* periplasm. Transcription was terminated using a dual transcription terminator *rrnB t1t2.* The *lacIq* gene encoded the constitutively expressed Lac I repressor protein. This repressed transcription from the tac promoter until de-repression was induced by the presence of allolactose or IPTG. The origin of replication used was p15A, which maintained a low copy number. The plasmid contained a tetracycline resistance gene for antibiotic selection.

The transformation of the strains was carried out using the method found in Chung C.T et al Transformation and storage of bacterial cells in the same solution. PNAS 86:2172-2175 (1989).

### Growth of mutated E. coli strains and expression of anti-TNF Fab' in mutated E. coli strains using high density fermentations

The strains produced in Example 2 were tested in fermentation experiments comparing growth and expression of an anti-TNFα Fab':

### Growth medium.

The fermentation growth medium was based on SM6E medium (described in Humphreys et al., 2002, Protein Expression and Purification, 26, 309-320) with 3.86 g/l NaH₂PO₄.H₂O and 112 g/l glycerol.

### Inoculum.

Inoculum cultures were grown in the same medium supplemented with 10 µg/ml tetracycline. Cultures were incubated at 30°C with agitation for approximately 22 hours.

**Fermentation.** Fermenters (2.5 litres total volume) were seeded with inoculum culture to 0.3-0.5 OD₆₀₀. Temperature was maintained at 30°C during the growth phase and was reduced to 25°C prior to induction. The dissolved oxygen concentration was maintained above 30% air saturation by variable agitation and airflow. Culture pH was controlled at 7.0 by automatic titration with 15% (v/v) NH₄OH and 10% (v/v) conc. H₂S0₄. Foaming was controlled by the addition of 10% (v/v) Struktol J673 solution (Schill and Seilacher). A number of additions were made at different stages of the fermentation. When biomass concentration reached approximately 40 OD₆₀₀, magnesium salts and NaH₂PO₄.H₂O were added. Further additions of NaH₂PO₄.H₂O were made prior to and during the induction phase to ensure phosphate was maintained in excess. When the glycerol present at the beginning of fermentation had depleted (approximately 75 OD₆₀₀) a continuous feed of 80% (w/w) glycerol was applied. At the same point in the fermentation an IPTG feed at 170µM was applied. The start of IPTG feeding was taken as the start of induction. Fermentations were typically run for 64-120 hours at glycerol feed rates (ranging between 0.5 and 2.5 ml/h).

**Measurement of biomass concentration and growth rate.** Biomass concentration was determined by measuring the optical density of cultures at 600 nm.

**Periplasmic Extraction.** Cells were collected from culture samples by centrifugation. The supernatant fraction was retained (at -20°C) for further analysis. The cell pellet fraction was resuspended to the original culture volume in extraction buffer (100 mM Tris-HCl, 10 mM EDTA; pH 7.4). Following incubation at 60°C for approximately 16 hours the extract was clarified by centrifugation and the supernatant fraction retained (at -20°C) for analysis. **Fab' quantification.** Fab' concentrations in periplasmic extracts and culture supernatants were determined by Fab' assembly ELISA as described in Humphreys et al., 2002, Protein Expression and Purification, 26, 309-320 and using Protein G hplc. A HiTrap Protein-G HP 1ml column (GE-Healthcare or equivalent) was loaded with analyte (approximately neutral pH, 30°C, 0.2um filtered) at 2ml/min, the column was washed with 20mM phosphate, 50mM NaCl pH 7.4 and then Fab' eluted using an injection of 50mM Glycine/HCl pH 2.7. Eluted Fab' was measured by A280 on a Agilent 1100 or 1200 HPLC system and quantified by reference to a standard curve of a purified Fab' protein of known concentration.

The present invention also provides a therapeutic or diagnostic composition comprising the antibody produced by the method of the present invention in combination with a pharmaceutically acceptable excipient, diluent or carrier.

The present invention also provides a process for preparation of a therapeutic or diagnostic composition comprising admixing the antibody produced by the method of the present invention together with a pharmaceutically acceptable excipient, diluent or carrier.

While this invention has been particularly shown and described with reference to preferred embodiments, it will be understood to those skilled in the art that various changes in form and detail may be made without departing from the scope of the invention as defined by the appendant claims.

### In vitro assay for protein disulphide isomerase activity

The disulphide isomerase activity of wild type or mutated DsbC proteins can be determined using a number of *in vitro* assays. The most well described assay follows the return of enzymatic activity to RNAse which has been re-oxidised from a reduced and denatured form i.e. follows enzymatically the folding of 'scrambled RNAse'. This assay is commonly called the 'scrambled RNAse assay' or 'sRNAse assay'. The preparation of suitably scrambled RNAse is decribed in 'Formation and isomerization of disulfide bonds in proteins: protein disulfide-isomerase, Hillson, D A; Lambert, N; Freedman, R B in Methods in Enzymology 107:281-94, 1984. Examples of the application of the scrambled RNAase assay include; Lyles and Gilbert, Biochem. J. 1991 30:613-619 and Lambert and Freedman Biochem. J. 1983 213:235-243 and references therein.

Alternatively isomerase activity can be measured using the refolding of misfolded (scrambled) IGF1 as followed by hplc analytical methods; Joly and Swartz Biochemistry 1997 36:10067-10072 and references therein.

Alternatively, isomerase activity can be measured using the refolding of scrambled insulin as followed by hplc analytical methods; Tang et al., Biochem J 1988 255: 451-455.

The enzymatic activity of disulphide isomerases has also been measured using the oxidative refolding of reduced proteins such RNAse and insulin (Lang and Schmid Nature 1988 331:453-455. Isomerase activity is more meaningfully assesed by performing such oxidative refolding studies under conditions where the redox buffers are such that reduced agents (e.g. glutathione) are in molar excess to oxidised agents (oxidised glutathione).

## Claims

1. A recombinant gram-negative bacterial cell comprising:
a. a recombinant polynucleotide encoding a mutated DsbC; and
b. a polynucleotide sequence encoding an antibody or an antigen binding fragment thereof specific for human TNFα.

2. A cell according to claim 1, wherein the antibody a heavy chain wherein the variable domain comprises a CDR having the sequence given in SEQ ID NO:1 for CDRH1, SEQ ID NO:2 or SEQ ID NO:7, for CDRH2 and SEQ ID NO:3 for CDRH3 and a light chain wherein the variable domain comprises a CDR having the sequence given in SEQ ID NO:4 for CDRL1, SEQ ID NO:5 for CDRL2 and SEQ ID NO:6 for CDRL3.

3. A cell according to claim 2, wherein the antibody comprises the light chain variable region sequence given in SEQ ID NO:8 and the heavy chain variable region given in SEQ ID N0:14.

4. A cell according to any of claims 1 to 3, wherein the antibody is a Fab fragment.

5. A cell according to claim 4, wherein the Fab fragment comprises a heavy chain having the sequence given in SEQ ID NO:21 and a light chain having the sequence given in SEQ ID NO:19.

6. A cell according any preceding claim, wherein the cell is isogenic to a wild-type bacterial cell except for the recombinant polynucleotide encoding said mutant DsbC and the polynucleotide sequence encoding the antibody or antigen binding fragment thereof specific for human TNFα.

7. A cell according to any preceding claim, wherein the cell is E. *coli.*

8. A cell according to any preceding claim, wherein the mutant DsbC has at least equivalent biological activity to wild-type DsbC.

9. A cell according to claim 8, wherein the mutant DsbC has greater biological activity than wild-type DsbC.

10. A cell according to any one of claims 1 to 9, wherein the mutation is in an active site of the DsbC, wherein said active site has the structure -CXXC-.

11. A cell according to claim 10, wherein XX represents any two amino acids with the proviso that XX does not represent GF, for example X and X are independently selected from one of the twenty natural amino acids that occurring in proteins, in particular independently selected from alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine and valine

12. A cell according to claim 11, wherein XX is TF, GF, HH, NY, SF, MF, VH, SH, RF, FA, GA, MA, GI, AV, PS, QA, SV, PR, PP, AL, PL, FL, TR, LL, VL, QL, LQ.

13. An expression vector comprising a recombinant polynucleotide encoding a mutant DsbC and an antibody or an antigen binding fragment thereof specific for hTNFα.

14. An expression vector according to claim 13, which comprises a promoter which controls the expression of the recombinant polynucleotide encoding DsbC and the antibody or an antigen binding fragment thereof specific for human TNFα.

15. An expression vector according to claim 13 or claim 14, which comprises a dicistronic message for producing the antibody or antigen binding fragment thereof, in which the upstream cistron contains DNA coding for the light chain of the antibody and the downstream cistron contains DNA coding for the corresponding heavy chain, **characterised in that** the dicistronic message comprises a sequence selected from IGS1 (SEQ ID NO: 23), IGS2 (SEQ ID NO: 24), IGS3 (SEQ ID NO: 25) and IGS4 (SEQ ID NO: 26).

16. An expression vector according to any of claims 13 to 15, wherein the antibody or an antigen binding fragment thereof specific for human TNFα is as defined in any of claims 2 to 5.

17. A cell according to any one of claims 13 to 16, wherein the mutant DsbC has at least equivalent biological activity to wild-type DsbC.

18. A cell according to claim 17, wherein the mutant DsbC has greater biological activity than wild-type DsbC.

19. A cell according to any one of claims 13 to 18, wherein the mutation is in an active site of the DsbC, wherein said active site has the structure -CXXC-.

20. A cell according to claim 19, wherein XX represents NY, SF, TF, MF, GF, HH, VH, SH, RF, FA, GA, MA, GI or AV.

21. A cell according to claim 20, wherein XX represents NY, SF, TF, MF, GF, HH, VH or SH.

22. A cell according to any of claims 1 to 7, which comprises an expression vector as defined in any of claims 13 to 22.

23. A method for producing an antibody or an antigen binding fragment thereof specific for human TNFα comprising:
a. culturing a recombinant gram-negative bacterial cell as defined in any of claims 1 to 7 or 22 in a culture medium under conditions effective to express the antibody or the antigen binding fragment thereof specific for human TNFα and the recombinant polynucleotide encoding DsbC; and
b. recovering the antibody or an antigen binding fragment thereof specific for human TNFα from the periplasm of the recombinant gram-negative bacterial cell and/or the culture medium.

24. A method according to claim 23, wherein the expression of the polynucleotide sequence encoding the antibody or the antigen binding fragment thereof specific for human TNFα and the recombinant polynucleotide encoding DsbC is induced by adding an inducer to the culture medium.

25. A method according to claim 23 or claim 24, wherein the method further comprises separating the antibody or the antigen binding fragment thereof specific for human TNFα from DsbC.

26. A method according to any of claims 23 to 25, wherein the method further comprises a step of attaching an effector molecule to an amino acid at or towards the C-terminal end of the heavy chain and/or the light chain of the antibody.

27. A method according to claim 26, wherein the method comprises attaching to one of the cysteine residues at the C-terminal end of the heavy chain a lysyl-maleimide group wherein each amino group of the lysyl residue has covalently linked to it a methoxypoly(ethyleneglycol) residue having a molecular weight of about 20,000 Da.
